# EUROPEAN PATENT APPLICATION

(11) **EP 0 982 013 A1**
(43) Date of publication of application: **01.03.2000**
(21) Application number: 99202740.9
(22) Date of filing: 25.08.1999
(51) Int. Cl.: A61F 5/56

(54) **Device to prevent snoring**

(30) Priority: 28.08.1998 ES 9801832
(71) Applicant: Sanchez Pueyo, Lourdes, 50009 Zaragoza (ES)
(72) Inventor: Sanchez Pueyo, Lourdes, 50009 Zaragoza (ES)
(74) Representative: Ungria Lopez, Javier

(57) **Abstract**

Device to prevent snoring, of use for persons who snore, whereby the user puts same in position when going to bed, retaining it in position throughout the night, and which consists of a body (2) of a flexible, adaptable material, of a generally oval shape, which has some orifices (4) likewise of a generally oval shape, for air to pass in the inhale-exhale action. Likewise the body (2) may have in its central part a tab positioned horizontally with respect to its outside face.

## Description

### OBJECT OF THE INVENTION

The following invention, as indicated in the heading of this specification, concerns a device to prevent snoring, said device being defined by a flexible body generally oval in shape and relatively thin, inserted by the user when going to bed, between lips and teeth, such that the body in question has orifices for the passage of air in the reflex movement of inhaling and exhaling, avoiding the vibration of soft palate and uvula, and with this, snoring.

In addition the flexible body, generally oval in shape, inserted by the user between lips and teeth, may have, centrally located, a small orthogonal tab turned outwards, as the device is fitted in the mouth, said tab having an orifice.

Moreover, the flexible body that is located between lips and teeth can also have a slightly roughened inner surface to facilitate its positioning, and also a small central groove, with respect to its upper and/or lower perimeter, of variable length, in order to remain in relation to the frenum of the upper lip, collaborating with its positioning and fixation.

In addition, the flexible body includes a number of small orifices, triangular in section, that act as salivation deflectors.

Thus, the purpose of the device is to prevent the user from snoring, with the great benefit this represents, not only for those who live with him, but for the user himself, because those who snore, do not rest properly, because their own snoring can wake them up or not allow them to reach the REM phase of the sleep process, which results in tiredness and poor reflexes, because they do not sleep for the proper number of hours. This is further aggravated when the person who snores performs activities that require good attention, for example driving a vehicle or using dangerous machinery.

In addition, in the case of fat people, who are more prone to snore, more so when they sleep in a supine posture, this can lead to apnoeas that are on occasion prove particularly dangerous, even causing the death by asphyxia of the snoring person.

### FIELD OF APPLICATION

The device disclosed is applicable to all who snore, so that when fitted between lips and teeth, and being provided with a number of orifices in the reflex movement of inhaling and exhaling, the flow of air that passes is distributed adequately through nose and mouth and does not cause the soft palate and uvula to vibrate, which is the cause of snoring.

Thus, the person who snores, when going to bed, will fit the device between lips and teeth, which by its flexible shape and thinness, causes no discomfort to the user when fitted, and has the enormous and important benefit of preventing the user from snoring.

### BACKGROUND TO THE INVENTION

Everybody in general knows the problems that result from the fact that a person snores, not only for the person himself, but also for those who live with him, constituting a major social problem, because a large percentage of people snore.

It can be briefly pointed out that snoring comes about in the inhaling-exhaling reflex respiratory action, when the mouth is opened, because snoring does not usually occur in breathing through the nose or it is less intensive. Thus, when a person has a lack of buccal vestibule air, he opens his mouth to be able to complete ventilation, and the flow of air can cause the soft palate and uvula to vibrate, and for this reason snoring arises. Snoring therefore occurs more intensively when the person is lying in supine decubitus position.

Thus, although the person who snores may in certain cases not be at all aware of this, and consequently no sequelas arise, or it is thought there are none, it is also true that on many occasions the person who snores may wake himself up as a result of his own snores or because of his snoring he is incapable of reaching the REM phase in the sleep process, and does not achieve a suitable rest, for which reason it is commonly found that people who snore, have reduced reflex actions during the daytime, possibly becoming drowsy with the problem this brings with it, particularly when the person has to drive a vehicle or perform tasks with a certain degree of danger, since they could suffer or provoke accidents.

On the other hand, those closest to a person who snores and who live with him, come to suffer the consequences of the serious nuisance they have to tolerate, making daily family life very difficult.

Because of all this, many different ways have been tried to overcome the problem of all those who snore, but none of the solutions proposed has obtained satisfactory results.

Thus, among the methods employed, mention can be made of surgical approach, consisting of reducing the size of the soft palate and the uvula, so that the flow of air does not cause them to vibrate. This is an invasive method that is not acceptable for many people, as they do not wish to undergo a surgical operation for said condition, because it is not considered to be a real health problem. Also, there are times when the desired result is not forthcoming, whereby an operation with particularly painful after-effects, does not guarantee complete success.

Likewise, among the different methods used to prevent snoring, mention can be made of one that consists of an adhesive element with a flexible body that is stuck to the nose, the object being that the flexible body that carries the adhesive element provoke through its flexion the separation of the nasal alar cartilage, so that the nasal conduits are kept permanently open and facilitate the passage of air, in the inhaling and exhaling action.

In addition there are other devices, in the form of a clip, which are fitted inside the nasal cavities, and likewise tend to open the nasal alar cartilage at the lower end, however this provokes the simultaneous closing of the upper part of the nasal conduits, whereby the problem remains unresolved.

Lastly, there are homeopathic drugs that only work as mere anaesthetics to achieve a more fluid breathing, although in most cases these do not produce the desired effect either.

On the other hand, the title holder of the present file has Utility Model n^{o} U9601755 in his favour, in which the claim is made for a device to prevent snoring, said device comprising an arched body, provided with a number of orifices for the passage of air, in the reflex breathing action, the same being fitted between lips and teeth, covering approximately the incisors and canine teeth, it being possible to define the device by a unitary arched body or by a single unit consisting of an arched base body and two bodies that define the complete assembly.

One of the bodies that are joined to the base body is defined by a body which covers a large part of the surface area of the base body, it being possible to define the other as a longitudinal strip that has a central convexcurved protrusion or by a spiral filiform element.

### DESCRIPTION OF THE INVENTION

The present specification describes a device to prevent snoring, which is useful for people who snore, such that it is inserted by the user on going to bed, and kept in place all night long, said device consisting of a body of flexible and adaptable material, generally oval in shape, that has some orifices, also generally oval in shape, for the passage of air during inhaling-exhaling and a number of orifices, of triangular shape, that deflect the salivation, so that the adaptability of said device permits a perfect fit in the user's mouth, independent of their morphology.

The oval shaped orifices for the passage of air are aligned longitudinally to the body of generally oval shape, slightly below its longitudinal axis.

Likewise, the oval-shaped orifices for the passage of air may also lie parallel to one another, transversally to the body, positioned symmetrically with respect to its transversal axis.

Two of the triangularly shaped orifices, that deflect the saliva, are arranged with respect to the most remote ends of the oval body while the other two are near the lower side. Each triangular orifice of the two pairs described, are arranged in inverted position, to favour the discharge of saliva flow and its perfect circulation.

The external face of the body generally oval in shape, may have a centrally located tab in a horizontal position, said tab having an orifice for the passage of air exhaled through the nose, and also so the user can fit a tape or cord fixed to this, attached at the other end to an article of the user's clothing or placed around his neck, to avoid the user's psychological fear of possibly swallowing the device.

The base body, generally oval in shape, may present a slightly roughened surface on its inner face, that facilitates its perfect positioning and fixation, without movements that could detract its efficiency and make it uncomfortable, while obviating any discomfort to the gums.

The base body, generally oval in shape, may present with respect to the perimeter of its central upper part, a groove of variable length, that will coincide with the frenum of the user's upper lip, allowing a better positioning and centring.

Also, the base body, generally oval in shape, may also have grooves in both the upper and lower central part, that will coincide with the frenum of the user's upper and lower lips, respectively, achieving thereby a more anatomical shape and easier positioning and centring.

The base body, generally oval in shape, is fitted between the user's lips and teeth, rests with its inner, slightly roughened face, in contact with the teeth and gums, and with the horizontal tab that protrudes outwards between the lips.

To complete the description that will be given below, and to provide a better understanding of the characteristics of the invention, a sheet accompanies this specification containing figures, given for the purpose of illustration only and not limiting in any way, representing the most characteristic details of the invention.

### SHORT DESCRIPTION OF THE DESIGNS

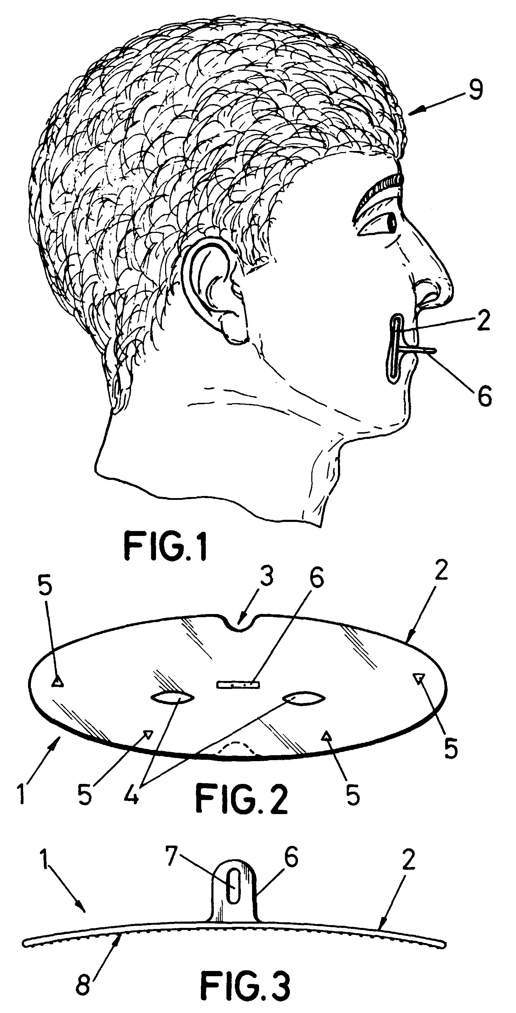

Figure 1. Shows a side view of a user wearing the selected device to prevent snoring, fitted between lips and teeth, where the tab can be observed, centred and horizontal, that protrudes between his lips to outside the mouth.
Figure 2. Shows a front view of the device to prevent snoring, defined by a laminar body, generally oval in shape, where the pair of orifices for the passage of inhaled and exhaled air can be seen, and also the tab fitted in the centre and the salivation deflector triangular orifices.
Figure 3. Shows a top view of the device to prevent snoring, defined by a laminar body, generally oval in shape, where the orthogonal tab that protrudes between the lips is observed, and which has an orifice for the passage of air, exhaled through the nose, and to allow the user to fasten a possible tape or cord.

### DESCRIPTION OF A PREFERRED EMBODIMENT

In view of the above mentioned figures and in accordance with the numeration adopted, it can be observed how device 1 to prevent snoring, is defined by a body 2, generally oval in shape, that the user 9 will fit between lips and teeth, such that to facilitate its correct positioning, body 2 has a groove 3 with respect to the upper central part which when fitted, coincides with the upper lip frenum. The device may also have a similar groove at the bottom, to be fitted with respect to the frenum of the lower lip, achieving a more anatomical shape and easier fitting. This groove 2 may be of variable arc and length.

Body 2 has measurements such that although the user, in the sleep phase, is lying in supine decubitus and has his mouth open, it is impossible for the device to slip inside the mouth cavity and for him to suffer an accident or swallow it, because its side parts are perfectly placed between teeth and lips.

Likewise body 2 also has orifices 4, generally oval in shape, and that can be arranged horizontally or vertically, and through which the air passes in the reflex action of inhaling and exhaling. In addition, body 2 presents a number of orifices 5, generally triangular in shape, that act as salivation deflectors, allowing a correct passage of saliva, without causing the least discomfort to the user.

In addition, body 2 may have a tab 6 placed horizontally with respect to the central part of its outside face, so that when the user puts device 1 in place, it lies between his lips towards the outer part, and will have an orifice 7.

This orifice 7 has various functions and thus allows the passage of air exhaled through the nose and also a tape or cord can be tied hereto, at the wish of the user, the other end being attached to the user's clothing, or else worn round his neck, should he be in fear of swallowing the device, even though this is not possible.

Also the inner face 8 of body 2 may have a slightly rough surface that collaborates in its positioning and fixing in place, and in no way causes discomfort to the user's gums.

Because of the anatomic shape of the device to prevent snoring, it is not at all uncomfortable to wear because it will only be used when the user goes to bed, without causing him any discomfort and also due to its size, as mentioned, it does not present any danger of being accidentally swallowed, but also for the user's greater tranquillity, the device object of the invention may be tied round his neck or to some item of clothing by an external element, for example a cord or tape, tied to the orifice in the tab.

Body 2 may be made in different materials and has a flexibility that allows it to be adapted over the user's gums without any problem, regardless of his specific morphology.

## Claims

1. DEVICE TO PREVENT SNORING, that is useful for people who snore, so that the user fits it in place before going to bed and wears it throughout the night, characterised in that the device (1) includes a body (2) of flexible and adaptable material, generally oval in shape, that presents orifices (4) also generally oval in shape, for the passage of air in inhaling-exhaling actions, and a series of orifices (5) being triangular in shape to deflect salivation.

2. DEVICE TO PREVENT SNORING, in accordance with claim 1, characterised in that the orifices (4) that are oval in shape for the passage of air, are longitudinally aligned to the body (2) generally oval in shape slightly below its longitudinal axis.

3. DEVICE TO PREVENT SNORING, in accordance with claim 1, characterised in that the orifices (4) oval in shape for the passage of air, are parallel to one another, transversal to the body (2), positioned symmetrically with respect to its transversal axis.

4. DEVICE TO PREVENT SNORING, in accordance with claim 1, characterised in that two of the orifices (5) that are triangular in shape, that deflect the saliva, are arranged with respect to the remotest extremities of the oval body (2) and the other two near the lower side, where each triangular orifice (5) of the two pairs described is arranged in inverted position.

5. DEVICE TO PREVENT SNORING, in accordance with claim 1, characterised in that the body (2) generally oval in shape can have a centred and horizontally positioned tab (6) with respect to its external face, having an orifice (7).

6. DEVICE TO PREVENT SNORING, in accordance with claim 1, characterised in that the body (2), generally oval in shape, can have a slightly roughened surface on its inner face (8).

7. DEVICE TO PREVENT SNORING, in accordance with claim 1, characterised in that the body (2) generally oval in shape may present a groove (3) with respect to the upper central part, that will coincide with the frenum of the user's upper lip.

8. DEVICE TO PREVENT SNORING, in accordance with claim 1, characterised in that the body (2) generally oval in shape, may have two grooves with respect to the upper and lower central part, that will coincide with the frena of the user's upper and lower lips, respectively.

9. DEVICE TO PREVENT SNORING, in accordance with the above claims, characterised in that in its placement between the user's teeth and lips, the body (2) has a slightly roughened inner face in contact with the teeth and gums, and with the horizontal tab (6), protruding outwards between the lips.
